# EUROPEAN PATENT SPECIFICATION

(11) **EP 4 516 269 B1**
(45) Date of publication and mention of the grant of the patent: **28.01.2026**
(21) Application number: 24193788.7
(22) Date of filing: 09.08.2024
(51) Int. Cl.: A61F 2/46

(54) **MENISCAL DOVETAIL SLOT CONVERTER**
MENISKUS-SCHWALBENSCHWANZSCHLITZWANDLER
CONVERTISSEUR MÉNISCAL À RAINURE EN QUEUE D'ARONDE

(30) Priority: 21.08.2023 US 202363520684 P
(43) Date of publication of application: 05.03.2025
(73) Proprietor: Arthrex, Inc., Naples, FL 34108-1945 (US)
(72) Inventor: CANNON, Bryan, Miami, 33176 (US); BENEDICT, Robert, Naples, 34119 (US); NOBLE, Shane, Naples, 34109 (US); COLE, Brian J., Chicago, 60612 (US)
(74) Representative: Lohr, Jöstingmeier & Partner Patent- und Rechtsanwälte mbB

(56) References cited:
- US-A1- 2013 096 680
- US-A1- 2016 270 933
- US-A1- 2022 117 758
- US-A1- 2023 218 403

## Description

### BACKGROUND

Meniscal allograft transplantation is a common technique for total replacement of a torn lateral or medial meniscus. Meniscal allografts have been found to be a feasible alternative in the effort to limit sequelae of arthritis that can occur with meniscal excision. One common type of meniscal allograft transplantation utilizes a trapezoidal bone block commonly known as a dovetail meniscal allograft. However, some surgeons prefer an alternative surgical technique requiring a meniscal allograft in the shape of a rectangular slot. As such, an improved system that converts the dovetail shape of the prepared meniscal allograft into a rectangular slot is desirable.

US 2022/117758 Al discloses a graft preparation station for repairing bone defects.

### SUMMARY

The disclosure herein includes a meniscal allograft dovetail slot converter.

The invention is defined in claim 1.

Other aspects, advantages, and alternatives, will become apparent to those of ordinary skill in the art by reading the following detailed description, with reference where appropriate to the accompanying drawings.

### BRIEF DESCRIPTION OF THE DRAWINGS

Figure 1 is a perspective view of an example device for use in a meniscal allograft transplantation procedure.
Figure 2 is a top view of the example device of Figure 1.
Figure 3 is a perspective view of the device of Figure 1 with a meniscal allograft positioned thereon.
Figure 4 is a perspective view of the device of Figure 1 with a meniscal allograft positioned therein.

### DETAILED DESCRIPTION

With reference to the Figures, Figures 1-4 illustrate a device 100 for use in a meniscal allograft transplantation procedure. As shown in Figures 1-4, the device 100 includes a base 102. The device 100 also includes a first sidewall 104 extending vertically from the base 102 and a second sidewall 106 extending vertically from the base 102. The second sidewall 106 is spaced away from the first sidewall 104 at a fixed distance. The device 100 also includes a third sidewall 108 extending vertically from the base 102. The third sidewall 108 is coupled to the first sidewall 104 and the second sidewall 106. In one example, the third sidewall 108 is perpendicular to both the first sidewall 104 and the second sidewall 106. The device 100 also includes a locking member 110 coupled to the second sidewall 106. The device 100 also includes a removable cutting guide 112 including a slotted through-hole 114. The removable cutting guide 112 is configured to be removably coupled to the first sidewall 104 and the second sidewall 106.

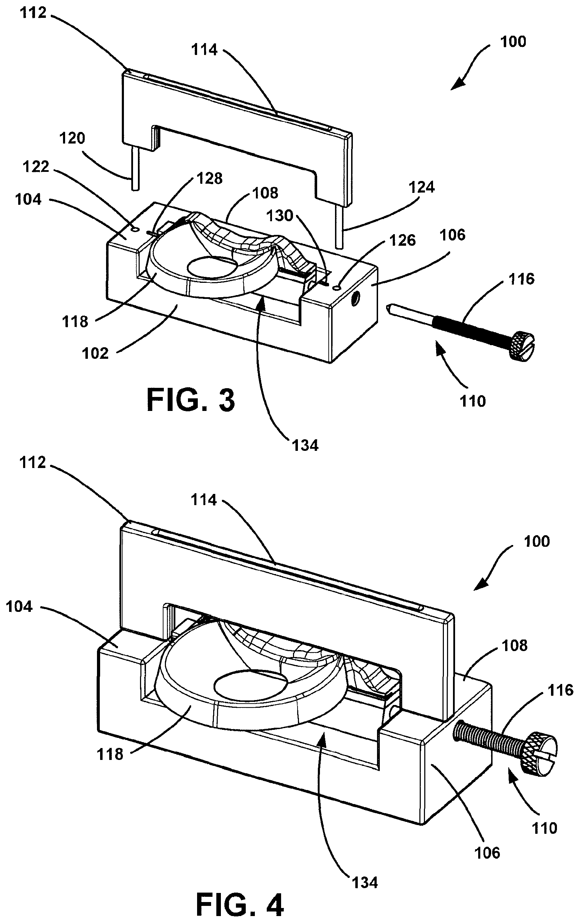

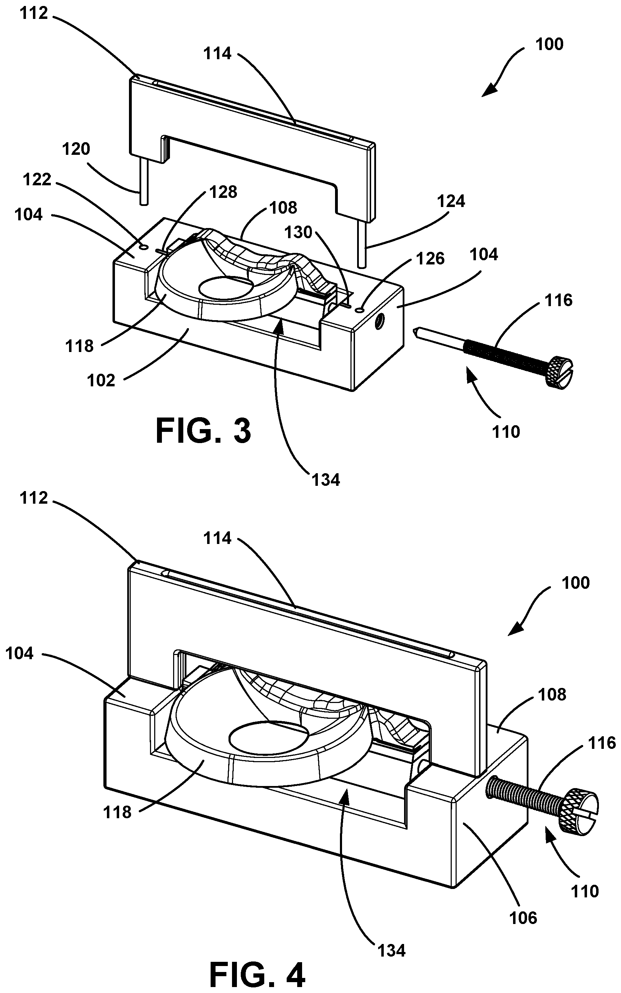

In one example, as shown in Figures 3-4, the locking member 110 comprises a threaded rod 116. In such an example, rotation of the threaded rod 116 in a first direction moves the locking member 110 closer to the first sidewall 104, and rotation of the threaded rod 116 in a second direction moves the locking member 110 further from the first sidewall 104. In one example, a longitudinal axis of the threaded rod 116 is offset from the slotted through-hole 114. Such an arrangement ensures that when a saw is positioned through the slotted through-hole 114 to trim the meniscal allograft 118, the saw does not contact the threaded rod 116. In one particular example, the threaded rod 116 comprises a set screw with a first portion that is threaded and a second portion that is not threaded.

In one example, the device 100 includes a first locking member coupled to the first sidewall and a second locking member coupled to the second sidewall. In another example, the device includes a single locking member coupled to the first sidewall or the second sidewall.

According to the invention, the removable cutting guide 112 includes a first pin 120 configured to be positioned in a first hole 122 in the first sidewall 104, and the removable cutting guide 112 includes a second pin 124 configured to be positioned in a second hole 126 in the second sidewall 106.

In one example, a length of the slotted through-hole 114 is greater than the fixed distance between the first sidewall 104 and the second sidewall 106. In one such example, the first sidewall 104 includes a first groove 128 and the second sidewall 106 includes a second groove 130. The first groove 128 and the second groove 130 are vertically aligned with the slotted through-hole 114 when the removable cutting guide 112 is removably coupled to the first sidewall 104 and the second sidewall 106. In one such example, a distance from a bottom of the first groove 128 to a bottom of the second groove 130 is equal to the length of the slotted through-hole.

In one example, the slotted through-hole 114 comprises a first slotted through-hole, and the base 102 includes a second slotted through-hole 132. In such an example, the second slotted through-hole 132 is vertically aligned with the first slotted through-hole 114 when the removable cutting guide 112 is removably coupled to the first sidewall 104 and the second sidewall 106. Such an arrangement may help ensure that, when in use, a saw goes all the way through the meniscal allograft 118 positioned in the device 100. The second slotted through-hole 132 further enables easy removal of debris from the device 100 after use, since the debris can be flushed out through the second slotted through-hole 132.

In another example, the second slotted through-hole 132 is a groove in the base 102 that does not pass all the way through the base 102. Such a groove is vertically aligned with the slotted through-hole 114 when the removable cutting guide 112 is removably coupled to the first sidewall 104 and the second sidewall 106. Such an arrangement may help ensure that, when in use, a saw goes all the way through the meniscal allograft 118 positioned in the device 100.

In one example, the device 100 further includes an opening 134 opposite the third sidewall 108. The opening 134 may define an area to receive a meniscal allograft 118 when in use.

As described above, meniscal allografts have been found to be a feasible alternative in the effort to limit sequelae of arthritis that can occur with meniscal excision. Simplified graft preparation and recipient tibia preparation, to allow for the transplant to be positioned anatomically and anchored with reliable fixation, is the ultimate goal of the procedure. The meniscal allograft includes two components: the lateral meniscus allograft and the bone bridge. Current meniscal allograft implants are prepared for surgery with the bone bridge in the shape of a dovetail. Some surgeons have preference on alternative surgical technique requiring the bone bridge of the meniscal allograft to be in the shape of a rectangular slot. The device 100 described above creates a new guided approach to convert the dovetail shape of the prepared bone bridge of the meniscal allograft into a rectangular slot. As most meniscal allografts are delivered with the bone bridge portion in the shape of a dovetail, the device 100 described above may assist in inventory management since the dovetail bone bridge can easily be transformed into a rectangular bone bridge.

Thus, in use, the surgeon prepares a rectangular slot in the tibia of the patient. Next, the surgeon positions a meniscal allograft 118 with a dovetail-shaped bone bridge on the base 102 of the device 100 between the first sidewall 104 and the second sidewall 106. The locking member 110 is used to secure the meniscal allograft 118 to the device 100. The variability of the screw length of the locking member 110 accommodates all different sized allografts. Next, the surgeon positions the removable cutting guide 112 onto the device 100. Once the removable cutting guide 112 is in place, the surgeon makes a cut with a saw through the slotted through-hole 114 to trim the dovetail-shaped bone bridge of the meniscal allograft 118 into a rectangular slot. The surgeon then positions the rectangular bone bridge of the meniscal allograft 118 into the prepared rectangular slot in the tibia. Next, as the meniscal allograft is positioned, the surgeon leads the graft passing suture out the posterior lateral capsule via a standard inside/out meniscal suturing technique to thereby provide peripheral graft fixation to the capsular rim.

It should be understood that arrangements described herein are for purposes of example only. As such, those skilled in the art will appreciate that other arrangements and other elements (e.g. machines, interfaces, functions, orders, and groupings of functions, etc.) can be used instead, and some elements may be omitted altogether according to the desired results. Further, many of the elements that are described are functional entities that may be implemented as discrete or distributed components or in conjunction with other components, in any suitable combination and location, or other structural elements described as independent structures may be combined.

While various aspects and examples have been disclosed herein, other aspects and examples will be apparent to those skilled in the art. The various aspects and examples disclosed herein are for purposes of illustration and are not intended to be limiting, with the true scope of the invention being defined by the appended claims. It is also to be understood that the terminology used herein is for the purpose of describing particular examples only, and is not intended to be limiting.

Example methods and systems are described herein. It should be understood that the words "example," "exemplary," and "illustrative" are used herein to mean "serving as an example, instance, or illustration." Any example or feature described herein as being an "example," being "exemplary," or being "illustrative" is not necessarily to be construed as preferred or advantageous over other examples or features. The examples described herein are not meant to be limiting. It will be readily understood that the aspects of the present disclosure, as generally described herein, and illustrated in the figures, can be arranged, substituted, combined, separated, and designed in a wide variety of different configurations, all of which are explicitly contemplated herein.

Furthermore, the particular arrangements shown in the Figures should not be viewed as limiting. It should be understood that other examples may include more or less of each element shown in a given Figure. Further, some of the illustrated elements may be combined or omitted. Yet further, an example may include elements that are not illustrated in the Figures.

In the following description, numerous specific details are set forth to provide a thorough understanding of the disclosed concepts, which may be practiced without some or all of these particulars. In other instances, details of known devices and/or processes have been omitted to avoid unnecessarily obscuring the disclosure. While some concepts will be described in conjunction with specific examples, it will be understood that these examples are not intended to be limiting.

As used herein, "coupled" means associated directly as well as indirectly. For example, a member A may be directly associated with a member B, or may be indirectly associated therewith, e.g., via another member C. It will be understood that not all relationships among the various disclosed elements are necessarily represented.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

Reference herein to "one embodiment" or "one example" or "an example" means that one or more feature, structure, or characteristic described in connection with the example is included in at least one implementation. The phrases "one embodiment" or "one example" or "an example" in various places in the specification may or may not be referring to the same example.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

By the term "about," "approximately," or "substantially" with reference to amounts or measurement values described herein, it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide. For example, in one embodiment, the term "about" can refer to ± 5% of a given value.

## Claims

1. A device (100) for preparing a bone graft, the device comprising:
a base (102);
a first sidewall (104) extending vertically from the base (102);
a second sidewall (106) extending vertically from the base (102), wherein the second sidewall (106) is spaced away from the first sidewall (104) at a fixed distance;
a third sidewall (108) extending vertically from the base (102), wherein the third sidewall (106) is coupled to the first sidewall (104) and the second sidewall (106);
a locking member (110) coupled to the second sidewall (106); and
a removable cutting guide (112) including a slotted through-hole (114), wherein the removable cutting guide (112) is configured to be removably coupled to the first sidewall (104) and the second sidewall (106),
**characterized in that**
the removable cutting guide (112) includes a first pin (120) configured to be positioned in a first hole (122) in the first sidewall (104), and wherein the removable cutting guide (112) includes a second pin (124) configured to be positioned in a second hole (126) in the second sidewall (106).

2. The device (100) of claim 1, wherein the locking member (110) comprises a threaded rod (116), and wherein rotation of the threaded rod (116) in a first direction moves the locking member (110) closer to the first sidewall (104), and wherein rotation of the threaded rod (116) in a second direction moves the locking member (110) further from the first sidewall (104).

3. The device (100) of claim 2, wherein a longitudinal axis of the threaded rod (116) is offset from the slotted through-hole (114).

4. The device (100) of any one of claims 1-3, wherein the third sidewall (106) is perpendicular to both the first sidewall (104) and the second sidewall (106).

5. The device (100) of any one of claims 1-4, wherein a length of the slotted through-hole (114) is greater than the fixed distance between the first sidewall (104) and the second sidewall (106).

6. The device (100) of claim 5, wherein the first sidewall (104) includes a first groove (128), wherein the second sidewall (106) includes a second groove (130), and wherein the first groove (128) and the second groove (130) are vertically aligned with the slotted through-hole (114) when the removable cutting guide (112) is removably coupled to the first sidewall (104) and the second sidewall (106).

7. The device (100) of claim 6, wherein a distance from a bottom of the first groove (128) to a bottom of the second groove (130) is equal to the length of the slotted through-hole (114).

8. The device (100) of any one of claims 1-7, wherein the base (102) includes a third groove, and wherein the third groove is vertically aligned with the slotted through-hole (114) when the removable cutting guide (112) is removably coupled to the first sidewall (104) and the second sidewall (106).

9. The device (100) of any one of claims 1-7, wherein the slotted through-hole (114) comprises a first slotted through-hole (114), wherein the base (102) includes a second slotted through-hole (132), and wherein the second slotted through-hole (132) is vertically aligned with the first slotted through-hole (114) when the removable cutting guide (112) is removably coupled to the first sidewall (104) and the second sidewall (106).

10. The device (100) of any one of claims 1-9, further comprising an opening (134) opposite the third sidewall (106).

## Patentansprüche

1. Vorrichtung (100) zur Vorbereitung eines Knochentransplantats, wobei die Vorrichtung Folgendes umfasst:
eine Basis (102);
eine erste Seitenwand (104), die sich vertikal von der Basis (102) erstreckt;
eine zweite Seitenwand (106), die sich vertikal von der Basis (102) erstreckt, wobei die zweite Seitenwand (106) in einem festen Abstand von der ersten Seitenwand (104) beabstandet ist;
eine dritte Seitenwand (108), die sich vertikal von der Basis (102) erstreckt, wobei die dritte Seitenwand (106) mit der ersten Seitenwand (104) und der zweiten Seitenwand (106) gekoppelt ist;
ein Verriegelungselement (110), das mit der zweiten Seitenwand (106) gekoppelt ist; und
eine entfernbare Schneidführung (112), die ein schlitzförmiges Durchgangsloch (114) aufweist, wobei die entfernbare Schneidführung (112) so konfiguriert ist, dass sie entfernbar mit der ersten Seitenwand (104) und der zweiten Seitenwand (106) gekoppelt ist,
**dadurch gekennzeichnet, dass**
die entfernbare Schneidführung (112) einen ersten Stift (120) aufweist, der konfiguriert ist, in einem ersten Loch (122) in der ersten Seitenwand (104) positioniert zu werden, und wobei die entfernbare Schneidführung (112) einen zweiten Stift (124) aufweist, der konfiguriert ist, in einem zweiten Loch (126) in der zweiten Seitenwand (106) positioniert zu werden.

2. Vorrichtung (100) nach Anspruch 1, wobei das Verriegelungselement (110) eine Gewindestange (116) umfasst, und wobei eine Drehung der Gewindestange (116) in einer ersten Richtung das Verriegelungselement (110) näher an die erste Seitenwand (104) bewegt, und wobei eine Drehung der Gewindestange (116) in einer zweiten Richtung das Verriegelungselement (110) weiter von der ersten Seitenwand (104) weg bewegt.

3. Vorrichtung (100) nach Anspruch 2, wobei eine Längsachse der Gewindestange (116) von dem schlitzförmigen Durchgangsloch (114) versetzt ist.

4. Vorrichtung (100) nach einem der Ansprüche 1-3, wobei die dritte Seitenwand (106) sowohl zu der ersten Seitenwand (104) als auch zu der zweiten Seitenwand (106) senkrecht ist.

5. Vorrichtung (100) nach einem der Ansprüche 1-4, wobei eine Länge des schlitzförmigen Durchgangslochs (114) größer als der feste Abstand zwischen der ersten Seitenwand (104) und der zweiten Seitenwand (106) ist.

6. Vorrichtung (100) nach Anspruch 5, wobei die erste Seitenwand (104) eine erste Nut (128) aufweist, wobei die zweite Seitenwand (106) eine zweite Nut (130) aufweist, und wobei die erste Nut (128) und die zweite Nut (130) vertikal mit dem schlitzförmigen Durchgangsloch (114) ausgerichtet sind, wenn die entfernbare Schneidführung (112) entfernbar mit der ersten Seitenwand (104) und der zweiten Seitenwand (106) gekoppelt ist.

7. Vorrichtung (100) nach Anspruch 6, wobei ein Abstand von einem Boden der ersten Nut (128) zu einem Boden der zweiten Nut (130) gleich der Länge des schlitzförmigen Durchgangslochs (114) ist.

8. Vorrichtung (100) nach einem der Ansprüche 1-7, wobei die Basis (102) eine dritte Nut aufweist, und wobei die dritte Nut vertikal mit dem schlitzförmigen Durchgangsloch (114) ausgerichtet ist, wenn die entfernbare Schneidführung (112) entfernbar mit der ersten Seitenwand (104) und der zweiten Seitenwand (106) gekoppelt ist.

9. Vorrichtung (100) nach einem der Ansprüche 1-7, wobei das schlitzförmige Durchgangsloch (114) ein erstes schlitzförmiges Durchgangsloch (114) aufweist, wobei die Basis (102) ein zweites schlitzförmiges Durchgangsloch (132) umfasst, und wobei das zweite schlitzförmige Durchgangsloch (132) vertikal mit dem ersten schlitzförmigen Durchgangsloch (114) ausgerichtet ist, wenn die entfernbare Schneidführung (112) entfernbar mit der ersten Seitenwand (104) und der zweiten Seitenwand (106) gekoppelt ist.

10. Vorrichtung (100) nach einem der Ansprüche 1-9, die ferner eine Öffnung (134) gegenüber der dritten Seitenwand (106) umfasst.

## Revendications

1. Dispositif (100) pour préparer une greffe osseuse, le dispositif comprenant :
une base (102) ;
une première paroi latérale (104) s'étendant verticalement à partir de la base (102) ;
une deuxième paroi latérale (106) s'étendant verticalement à partir de la base (102), dans lequel la deuxième paroi latérale (106) est espacée de la première paroi latérale (104) à une distance fixe ;
une troisième paroi latérale (108) s'étendant verticalement à partir de la base (102), dans lequel la troisième paroi latérale (106) est couplée à la première paroi latérale (104) et à la deuxième paroi latérale (106) ;
un élément de verrouillage (110) couplé à la deuxième paroi latérale (106) ; et
un guide de coupe amovible (112) comprenant un trou traversant fendu (114), dans lequel le guide de coupe amovible (112) est configuré pour être couplé de manière amovible à la première paroi latérale (104) et à la deuxième paroi latérale (106),
**caractérisé en ce que**
le guide de coupe amovible (112) comprend une première broche (120) configurée pour être positionnée dans un premier trou (122) dans la première paroi latérale (104), et dans lequel le guide de coupe amovible (112) comprend une deuxième broche (124) configurée pour être positionnée dans un deuxième trou (126) dans la deuxième paroi latérale (106).

2. Dispositif (100) selon la revendication 1, dans lequel l'élément de verrouillage (110) comprend une tige filetée (116), et dans lequel la rotation de la tige filetée (116) dans une première direction déplace l'élément de verrouillage (110) plus près de la première paroi latérale (104), et dans lequel la rotation de la tige filetée (116) dans une deuxième direction déplace l'élément de verrouillage (110) plus loin de la première paroi latérale (104).

3. Dispositif (100) selon la revendication 2, dans lequel un axe longitudinal de la tige filetée (116) est décalé par rapport au trou traversant fendu (114).

4. Dispositif (100) selon l'une quelconque des revendications 1 à 3, dans lequel la troisième paroi latérale (106) est perpendiculaire à la fois à la première paroi latérale (104) et à la deuxième paroi latérale (106).

5. Dispositif (100) selon l'une quelconque des revendications 1 à 4, dans lequel une longueur du trou traversant fendu (114) est supérieure à la distance fixe entre la première paroi latérale (104) et la deuxième paroi latérale (106).

6. Dispositif (100) selon la revendication 5, dans lequel la première paroi latérale (104) comprend une première rainure (128), dans lequel la deuxième paroi latérale (106) comprend une deuxième rainure (130), et dans lequel la première rainure (128) et la deuxième rainure (130) sont alignées verticalement avec le trou traversant fendu (114) lorsque le guide de coupe amovible (112) est couplé de manière amovible à la première paroi latérale (104) et à la deuxième paroi latérale (106).

7. Dispositif (100) selon la revendication 6, dans lequel une distance depuis un fond de la première rainure (128) jusqu'à un fond de la deuxième rainure (130) est égale à la longueur du trou traversant fendu (114).

8. Dispositif (100) selon l'une quelconque des revendications 1 à 7, dans lequel la base (102) comprend une troisième rainure, et dans lequel la troisième rainure est alignée verticalement avec le trou traversant fendu (114) lorsque le guide de coupe amovible (112) est couplé de manière amovible à la première paroi latérale (104) et à la deuxième paroi latérale (106).

9. Dispositif (100) selon l'une quelconque des revendications 1 à 7, dans lequel le trou traversant fendu (114) comprend un premier trou traversant fendu (114), dans lequel la base (102) comprend un deuxième trou traversant fendu (132), et dans lequel le deuxième trou traversant fendu (132) est aligné verticalement avec le premier trou traversant fendu (114) lorsque le guide de coupe amovible (112) est couplé de manière amovible à la première paroi latérale (104) et à la deuxième paroi latérale (106).

10. Dispositif (100) selon l'une quelconque des revendications 1 à 9, comprenant en outre une ouverture (134) opposée à la troisième paroi latérale (106).
